# EUROPEAN PATENT APPLICATION

(11) **EP 4 702 998 A1**
(43) Date of publication of application: **04.03.2026**
(21) Application number: 24196618.3
(22) Date of filing: 27.08.2024
(51) Int. Cl.: A61M 5/24, A61M 5/31

(54) **LOCK MECHANISM FOR A RESERVOIR HOLDER OF A DRUG DELIVERY DEVICE**

(71) Applicant: Ypsomed AG, 3401 Burgdorf (CH)
(72) Inventor: Bosshard, Simon Martin, 3324 Hindelbank (CH); Kalbermatter, Gabriel, 3400 Burgdorf (CH); Schrul, Christian, 3414 Oberburg (CH); Schindler, Sarah, 3018 Bern (CH); von Mühlenen, Beat, 3043 Uetligen (CH); Glauser, Oliver, 3600 Thun (CH)

(57) **Abstract**

The invention relates to a lock mechanism (10) for locking a distal reservoir holder (20) to a proximal part (6) of an injection device (1). The reservoir holder (20) comprises a first longitudinal guiding element (21), a proximally directed first sloped surface (31) and a distally directed first stop surface (35). The proximal part (6) comprises a second longitudinal guiding element (62) and a rotatable lock member (40) which includes a distally directed second sloped surface (51) and a proximally directed second stop surface (55). During an attachment movement of the reservoir holder (20) the first and second guiding element (21, 62) are coupled to each other and the first and second sloped surface (31, 51) are pressed against each other and subsequently slide along each other thereby causing the lock member (40) from a start position towards a rotated position.

## Description

### FIELD OF THE INVENTION

The present invention relates to a lock mechanism for a drug delivery device for dispensing a liquid drug through an injection needle for injecting, delivering, administering, infusing or dispensing substances and/or liquids such as insulin, hormone preparations or vaccines. The lock mechanism comprises a reservoir holder for holding the reservoir with the liquid drug and a housing with a rotatable lock member. The reservoir holder includes a first sloped surface adapted to slide along the second sloped surface of the lock member to rotate the lock member relative to the housing.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may be manually selected by turning a dosage knob and observing the actual dialed dose from a dose window or display of the injection pen. A dose is dispensed by inserting the needle into a suited portion of human skin and by moving the piston manually or by pressing a release button of an automatic injection device. Automatic injection devices may comprise an electronic dose dial mechanism to automatically set a dose.

The reusable or semi-reusable delivery device usually comprise a housing accommodating a dose and dispensing mechanism and a reservoir holder adapted to hold a reservoir, e.g. a cartridge or a syringe, in place relative to the housing.

To releasably attach the reservoir holder to the housing numerous coupling systems have been developed. Well known mechanisms include screw connections and bayonet or bayonet-like connections.

WO 2008/074897 A1 discloses a coupling mechanism to releasably attach a cartridge holder with a cartridge to an injection pen housing. The cartridge holder comprises two oppositely arranged protrusion which fit into two corresponding grooves in a connecting structure in the device housing. The guide includes a sloped section and a subsequent circumferential section. To attach the cartridge holder to the housing the protrusions have to be inserted first into the sloped section and subsequently the cartridge holder is rotated relative to the housing such that the protrusion enters the circumferential section. The cartridge holder is thus locked to the housing by a bayonet-like connection.

US 2012238960 A1 discloses a cartridge holder with a coding protrusion in a coupling portion. In case an injection pen housing includes a corresponding groove to accommodate the coding protrusion the cartridge holder can be connected to the housing by the bayonet connection.

Such coupling mechanisms provide a reliable and releasable attachment of the reservoir holder to the housing to facilitate replacement of a reservoir. However, the coupling and attachment procedure often requires a linear as well as a subsequent rotational movement which often constitutes a barrier in particular for people with dexterity problems.

### DESCRIPTION OF THE INVENTION

It is an objective of the invention to provide attachment and locking of a reservoir holder to a housing of an injection device wherein the attachment procedure is easy and does not require complex movements.

This objective is achieved by a lock mechanism and a method for locking a reservoir holder to a housing according to the independent claims. Preferred embodiments are evident from the dependent claims.

The invention relates to a lock mechanism for locking a distal reservoir holder to a proximal part of an injection device. The injection device is adapted to dispense a liquid drug from a reservoir through an injection needle. The lock mechanism comprises the distal reservoir holder adapted to hold the reservoir in place relative to the proximal part and releasably attachable and lockable to the proximal part. The reservoir holder comprises
- a first longitudinal guiding element,
- a proximally directed first sloped surface and
- a distally directed first stop surface.

The lock mechanism further comprises the proximal part defining a longitudinal axis and comprising
- a housing adapted to accommodate a dose and dispensing mechanism for dispensing the liquid drug;
- a second longitudinal guiding element adapted to cooperate with the first longitudinal guiding element and
- a spring member adapted to bias the lock member;
- a lock member rotationally guided by the housing or a supporting structure such that a rotation of the lock member relative to the housing is allowed but a longitudinal movement relative to the housing is prevented. The lock member comprises
   i. a distally directed second sloped surface and
   ii. a proximally directed second stop surface.

During an attachment movement of the reservoir holder relative to the housing in proximal direction towards an attached and locked position to attach and lock the reservoir holder to the proximal part
- the first and second guiding element are coupled to each other and cooperate with each other therefore forming a linear guide and thereby allowing a longitudinal movement but preventing a rotation of the reservoir holder relative to the housing during the attachment movement,
- the first and second sloped surface are pressed against each other and subsequently slide along each other thereby causing the lock member to rotate relative to the housing from a lock member start position towards a lock member rotated position and
- the first stop surface is located distally to the second stop surface and spaced apart from the second stop surface in the longitudinal direction.

When the reservoir holder is in its attached and locked position the lock member is in the rotated position and the lock member rotation allows the first stop surface to pass the second stop surface in the longitudinal direction such that in the attached and locked position of the reservoir holder the first stop surface is located proximal to the second stop surface. The biased spring biases the lock member in its rotated position. Due to the lock member rotation the first and second stop surface are rotationally aligned to each other and can cooperate with each other thereby preventing a longitudinal movement of the reservoir holder in distal direction and locking the reservoir holder to the proximal part. That means the user can not remove the reservoir holder from the proximal part unless the lock mechanism is released.

The lock mechanism according to the invention holds and locks the reservoir holder to the proximal part of the injection device. That means the reservoir holder is held in place and after locking cannot be pulled away from the proximal part in distal direction. The injection device is then ready for an injection.

The lock mechanism allows for a simple and easy attachment and locking of the reservoir holder to the proximal device part as the user has to move the reservoir holder exclusively along the longitudinal axis to attach and lock the reservoir holder to the proximal part. Conventional mechanisms require a complex or combined locking movement to lock the reservoir holder to the proximal part or housing. Namely, well-known bayonet connections require a combined axial and rotational movement for locking the reservoir holder to a proximal part.

Furthermore, the locking mechanism according to the invention is similar to a ballpoint pen mechanism including a retraction mechanism with two cams and a guide pin and a spring providing a bistable system where in one position the writing tip is retracted and in the other the tip is extended. That means the user may already be used to such a mechanism which facilitates the handling.

Moreover, the lock mechanism according to the invention provides for a compact design as the mechanism requiring exclusively a longitudinal attachment movement does not require more space than known approaches with bayonet or screw type connections. Hence, the outer dimensions of the injection device remain the same with the lock mechanism according to the invention.

During the attachment and locking the reservoir holder is rotationally coupled to the proximal part by the first and second longitudinal guiding elements such that it can be exclusively moved along the longitudinal axis relative to the housing and relative rotation is prevented. The lock member is exclusively rotatable and cannot be moved longitudinally. When the reservoir holder is moved proximally towards its attached and locked position relative to the housing the first sloped surface slides along the second sloped surface of the lock member. As the lock member cannot move axially the lock member is forced to rotate out of its start or initial position towards a rotated position.

Due to the lock member rotation the first stop surface, which is distal to the second stop surface before attachment, can be moved proximal until the first stop surface is located proximal the second stop surface. That means the rotating lock member allows the first stop surface to move beyond or proximally to the second stop surface and/or to align the first stop surface to the second stop surface.

When the reservoir holder is in its attached and locked position the first and second stop surface are rotationally aligned and additionally are aligned to each other with respect to the longitudinal axis and are positioned opposite each other. As the reservoir holder stop surface is distally directed and the lock member stop surface is proximally directed the two stop surfaces can abut each other when the user tries to pull the reservoir holder back in distal direction (away from the housing and out of the locked position).

The spring member biases the lock member into its rotated position such that the lock member is reliably held in the rotated position. An unintentional release or rotation of the lock member is can thus be prevented. Moreover, the spring member ensures that the first and second stop surfaces are pressed against each other, and that the reservoir holder is held in a stable position relative to the proximal part of the injection device. That means there is no play and the reservoir holder does not move in its locking position relative to the proximal part.

When the reservoir holder is pulled distally along the longitudinal axis the sloped surfaces cannot cooperate due to the engagement of the first and second stop surfaces and thus the lock member cannot rotate. That means the lock member locks the reservoir holder to the proximal part of the injection device such that it cannot be removed by pulling it in distal direction. To release the reservoir holder the first and second stop surface have to be moved away from each other by a release movement as described in detail further below.

The housing defines a longitudinal axis and extends along the longitudinal axis. The housing may be sleeve-shaped. Further it may be adapted to accommodate a dose and dispensing mechanism of the injection device. The reservoir holder is releasably attachable and lockable to the proximal part and for example to the housing or a housing member. The reservoir holder has to be detached and removed from the proximal part to replace or to insert a new reservoir. The latter may be, for example, a cartridge with a needle mounting portion adapted to be coupled to a needle assembly before an injection. Alternatively, the reservoir may be a prefilled syringe having a syringe barrel and an injection needle permanently and non-releasably connected to the barrel.

The first sloped surface and the first stop surface are preferably located in a proximal portion of the reservoir holder and the second sloped surface and the second stop surface are preferably arranged in a distal portion of the housing to facilitate attachment and locking of the reservoir holder to the housing.

The first longitudinal guiding element may be, for example, a cam, protrusion or rib and the second longitudinal guiding element may be a corresponding groove or nut adapted to accommodate the first guiding element. Alternatively, the second longitudinal guiding element is a cam, protrusion or rib and the first guiding element is the groove or nut. The first and second guiding elements provide a longitudinal guide allowing a longitudinal movement but preventing a relative rotation between the reservoir holder and the housing.

The first and second sloped surfaces are arranged on the reservoir holder and the first and second stop surfaces are arranged on the lock member respectively. The first sloped surface and the first stop surface may be integrally formed in the reservoir holder as a monolithic part or alternatively, the surfaces may be arranged on an element fixedly connected to the reservoir holder. Accordingly, the second sloped surface and second stop surface may be integrally formed in the housing or may be arranged on an element fixedly connected to the housing.

The first and second sloped surfaces and/or the first and second stop surfaces may be part of a guiding member and in particular may be part of a cam control. Stated differently, the lock mechanism preferably includes a cam control which includes the first and second sloped surface and the first and second stop surface.

The reservoir holder may be initially in a non-attached separated state. When the user attaches the reservoir holder to the holder or a proximal part of the injection device the reservoir holder may be inserted into the housing or housing part or the housing may be inserted into the holder and subsequently, the reservoir holder and the housing are moved towards each other.

The reservoir holder is moved proximally relative to the housing until the holder is in an attached and locked position. That is the case after the lock member has been rotated into a rotated position. That means the reservoir holder is in its locked position if the first stop surface is located proximally to the second stop surface and the first and second stop surface are rotationally aligned to each other and are positioned such that the stop surfaces can engage each other.

The rotation of the lock member is required to let pass elements of the cam control and in particular the first and second stop surfaces such that the stop surfaces are rotationally and longitudinally aligned and positioned to each other after the lock member rotation and when the reservoir holder is in its attached position.

The injection device may be a reusable device or alternatively a semi-disposable (also named as semi-reusable) device. The housing and a dose and dispensing mechanism are preferably reusable and can be attached to a new reservoir via reservoir holder. The injection device is preferably a pen-shaped injection device, more preferably user actuated device devoid of any electric drive and motor.

The reservoir may be a container, cartridge (one or two chamber) adapted to contain the drug. The cartridge may be a non-refillable cartridge for single use. The cartridge includes a needle mounting portion where a needle assembly can be releasably attached to. Alternatively, the reservoir may be a prefilled syringe having a needle or cannula that is permanently attached to a first end of a syringe barrel that is sealed at the opposing end by the plunger.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the injection device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

In a preferred embodiment the reservoir holder includes a holder protrusion comprising on a proximal side or proximal face the first sloped surface and on a distal side or distal face the first stop surface. The protrusion comprising the sloped surface and stop surfaces provides a compact design as two functions are implemented in a single element. The first function is rotating the lock member (sloped surface) and the second function is locking the reservoir holder (stop surface).

Alternatively, the sloped surface and the stop surface are arranged on separate, distinct elements of the reservoir holder.

The lock member is biased in its initial position by a spring. The lock member may be operatively coupled to the spring either directly or indirectly via an intermediate member between the spring and the lock member.

Preferably, the rotation of the lock member from its start position into its rotated position is against a force of the spring member. When the lock member is in its rotated position the spring member biases the lock member such that the lock member is stable and is held in the rotated position. The spring ensures that the lock member remains in its start position or/and rotated position and is not unintentionally rotated out of the start position or/and is not unintentionally rotated away from its rotated position.

Moreover, the spring enables a return movement of the lock member in its start position or in a distinct holding position when the lock member has passed a trigger point. Alternatively, the lock member is rotated manually back and forth without spring force.

Preferably, the lock mechanism further includes a cam guided by a longitudinal guide, preferably provided by the housing or a supporting structure inside the housing. Due to the longitudinal guide the cam is exclusively movable along the longitudinal axis relative to the lock member and relative to the housing but is prevented from being rotated relative to the housing. Furthermore, the cam comprises an angled or sloped surface on its end and the lock member comprises an angled or sloped counter surface adapted to engage the angled cam surface.

By means of the spring the angled surface of the cam is pressed against the angled counter surface of the lock member. Due to the slope and as the lock member cannot move longitudinally but is exclusively rotatable the lock member is forced to rotate relative to the housing when the angled surface and the angled counter surface are pressed against each other until a arrowhead-shaped end of the cam is located between two oppositely angled lock member surfaces. That means the lock member is biased into its start position and to rotate the lock member out of its start position requires a force exceeding the spring force.

In a preferred embodiment first and second stop surfaces are sloped or inclined with respect to a direction perpendicular to the longitudinal direction. That means the two stop surfaces are oriented in an angle larger than 0° to a direction perpendicular to the longitudinal direction. Preferably, the first and second stop surfaces are oriented between 50 and 85 degree to the longitudinal axis or in an angle between 5 to 40 degree to an axis perpendicular to the longitudinal axis.

The sloped stop surfaces allow to reliably hold the reservoir holder in its locked position relative to the lock member and relative to the housing. That means the sloped stop surfaces may prevent an unintentional shifting or sliding of the first stop surface relative to the second stop surface because, for example, the slope is directed towards a wall or a stop element. The sloped surfaces may require an increased force to move the two surfaces relative to each other and thus to release the reservoir holder from its attached position.

Preferably, the lock member includes a guiding groove or the guiding groove is integrally formed in a surface of the lock member. The guiding groove may comprise on a distally faced wall the second sloped surface and on a proximally faced wall the second stop surface.

The guiding groove is thus part of a cam control between the reservoir holder and the lock member. That means the first sloped surface and the first stop surface can engage the lock member surfaces within the guiding groove and thus the holder is reliable guided by a predefined travel path formed by the guiding groove.

To release the reservoir holder from the proximal part the lock member may include a user actuating element which can be moved by the user to rotate the lock member relative to the housing. That means the user can rotate manually by a user force the lock member back from its rotated position towards its start position or towards another position.

The user actuating element may be, for example, an actuation lever, arm, knob, grip or protrusion.

Alternatively, the lock member may be rotated back by a spring biasing the lock member, for example, into an intermediate position.

As an alternative to the above-described guiding groove the lock member may include a lock protrusion comprising on a distal side the second sloped surface and on a proximal side the second stop surface. The second sloped surface and the second stop surface on one common element provides a compact design as two functions are implemented in one single element.

The lock protrusion is preferably L-shaped and includes a first portion extending circumferentially around the longitudinal axis and a second portion extending longitudinally. The first portion may include the second sloped surface on a distally directed face of the portion and the stop surface on a proximally directed face of the first portion. The second portion of the L-shaped protrusion may include the second axial surface extending along the longitudinal axis. The first axial surface may be arranged, for example, on a reservoir holder protrusion.

In a region where the two portions of the L-shaped protrusions are connected a recess or cavity may be formed which provides a stable position for a protrusion of the reservoir holder, if any.

Additionally, the lock member or proximal part of the injection device may include a cam control or guide for the holder protrusion. The cam control or guide may guide the holder protrusion during attachment. Namely, the holder protrusion may be guided and a proximal movement may be restricted such that the holder protrusion is forced to move in a holding position, in particular in the stable position provided by the L-shaped protrusion. That means the cam control or guide prevents the holder to be in an intermediate or unstable position.

Alternatively, the lock member may not include the above-mentioned protrusion but instead a guide, channel or groove adapted to accommodate and guide the holder protrusion during the attachment procedure.

The reservoir holder further preferably includes a first axial surface oriented parallel to the longitudinal axis and the lock member further includes a second axial surface oriented parallel to the longitudinal axis. When the lock member is in its offside position the first and second axial surfaces preferably cooperate with each other such that a further longitudinal movement of the reservoir holder relative to the lock member is allowed but a return rotation of the lock member towards its start position is prevented during the attachment movement.

When the reservoir holder is in a pre-attached position or at the very beginning of the attachment movement the first and second axial surfaces are not aligned and preferably circumferentially separated from each other. Preferably, when the lock member is in its start position the first and second axial surface are spatially separated in a circumferential direction. During rotation of the lock member from its start position into its rotated offside position the two axial surfaces are preferably moved towards each other.

In the lock member rotated position the two axial surfaces are positioned opposite each other with respect to the circumferential direction as well as the longitudinal direction. That means the two axial surfaces may abut each other and thus may block a rotation of the lock member. For that purpose, the first axial surface is preferably directed circumferentially towards the start position and the second axial surface of the lock member is directed circumferentially away from the start position.

The two axial surfaces longitudinally guide the movement of the reservoir holder relative to lock member and thus ensure that the lock member is not unintentionally rotated during the attachment movement. Depending on the position of the first and second stop surfaces the lock member may be required to remain in the rotated position until the reservoir holder is completely positioned relative to the lock member along the longitudinal axis. The first and second axial surfaces thus hold the rotated, and preferably biased, lock member in the rotated position away from the start position.

Preferably, the holder protrusion, if any, includes the first axial surface and the lock protrusion , if any, includes the second axial surface. In this embodiment the sloped surface, the stop surface and the axial surface are arranged on the holder protrusion and on the lock protrusion respectively. That may contribute to a compact design as all surfaces are arranged on a common element. Alternatively, the axial surfaces are on a separate element distinct from the holder protrusion or the lock protrusion, respectively.

In an alternative embodiment the lock member may be held by other means such as a protrusions and groove or may not be held at all, in particular if the lock member is not biased by a spring.

Preferably, when the reservoir holder is in the locked position the first and second axial surfaces have passed each other and are separated from each other in the longitudinal direction such that the lock member can be rotated towards the start position into the initial position or into a holding position. Stated differently, the two axial surfaces are adapted to guide the reservoir holder during the longitudinal attachment movement and before the reservoir holder reaches its locked position the two surfaces have passed each other and are longitudinally separated such that the lock member is no longer prevented from being rotated back towards its start position.

That means the first and second axial surfaces have a predefined length along the longitudinal axis to allow a back rotation once the two axial surfaces have passed each other during the attachment movement.

The rotation of the lock member brings second stop surface out of the travel path of the first stop surface when the reservoir holder is moved proximally, or the rotation brings the first stop surface to a position proximally to the second stop surface.

In an embodiment where the lock member is rotated back from the rotated position into its start position or into a holding position when the first and second sloped surfaces have passed each other the first and second stop surface are moved towards each other. When the reservoir holder is in its locked position the first and second stop surfaces are rotationally aligned and additionally are aligned to each other with respect to the longitudinal axis and are positioned opposite each other.

In a preferred embodiment the reservoir holder can be further in a proximal end position which is proximally to the attached position with respect to the housing and in which the stop surfaces are separated from each other in the longitudinal direction.

During a proximal release movement from the attached position towards the proximal end position the stop surfaces are separated from each other and the lock member is rotated out of its start position or out of its holding position.

In a subsequent longitudinally distal release movement of the reservoir holder relative to the lock member the second stop surface and a release surface of the lock member are pressed against each other which causes the surfaces to slide along each other thereby causing the lock member to be further rotated relative to the housing out of its holding position or out of its start position towards a release position such that the reservoir holder can be separated from the housing in a distal direction.

That means also for releasing the reservoir holder from the housing the user can simply push the reservoir holder further in proximal direction and subsequently pull the reservoir holder in distal direction without rotation relative to the housing. Hence, not only the attachment and locking but also the release is made by a longitudinal movement of the reservoir holder similar to a ballpoint pen with a two bistable positions.

The housing is preferably sleeve-shaped and the reservoir holder is preferably sleeve-shaped with an outer diameter smaller than the outer diameter of the housing such that the reservoir holder can be inserted into the housing at least with an end portion. That allows an easy attachment of the reservoir holder to the housing.

The invention relates further to an injection device comprising the lock mechanism as described above and a dose and dispensing mechanism accommodated inside the housing.

The injection device may be a reusable device including the reusable housing and the reservoir holder and a disposable reservoir held inside the reservoir holder.

Moreover, the invention relates to a method for locking a reservoir holder to a proximal part of a drug delivery device, the method comprises the steps of
a) Inserting a proximal end portion of the reservoir holder into a distal end portion of the proximal part which longitudinally guides the reservoir holder and prevents a rotation of the reservoir holder relative to the proximal part;
b) Moving the reservoir holder proximally relative to the housing towards a locked position;
c) Pressing, by the proximal movement, a reservoir holder first sloped surface against a lock member second sloped surface;
d) Rotating, by the sloped surfaces engagement, the lock member relative to a housing into a rotated position;
e) moving, due to the rotation, a reservoir holder first stop surface proximally to a lock member second stop surface;
f) Rotationally aligning, by the movement, the first stop surface and the second stop surface thereby preventing a longitudinal movement of the reservoir holder in distal direction and locking the reservoir holder to the housing.

### BRIEF DESCRIPTION OF THE DRAWINGS

The subject matter of the invention will be explained in more detail in the following text with reference to preferred exemplary embodiments which are illustrated in the attached drawings, in which:
- Fig.1: depicts a perspective view of an injection pen with a lock mechanism according to the invention;
- Fig.2: depicts the injection pen with the cartridge holder separated from a proximal part.
- Fig.3: depicts a detailed view of a proximal portion of the cartridge holder,
- Fig.4: depicts a detailed view of a distal portion of the lock member and housing;
- Fig. 5, 6: depict a side view of the cartridge holder and the lock member;
- Fig. 7-10: depict schematical views of attachment steps;
- Fig. 11-14: depict schematical views during the detachment of the cartridge holder from the proximal part;
- Fig. 15: depicts a perspective view of an injection pen with a lock mechanism according to a second embodiment of the present invention;
- Fig. 16: depicts a cartridge holder of the second embodiment;
- Fig. 17: depicts the lock member and proximal part without housing of the second embodiment
- Fig. 18-21: depict schematical views of an attachment steps of the second embodiment.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

In the present description the term "distal" refers to the side where the injection needle is located. This is on the left-hand side in the figures. The term "proximal" refers to the opposite side or rear end of the device which is on the right-hand side in the figures.

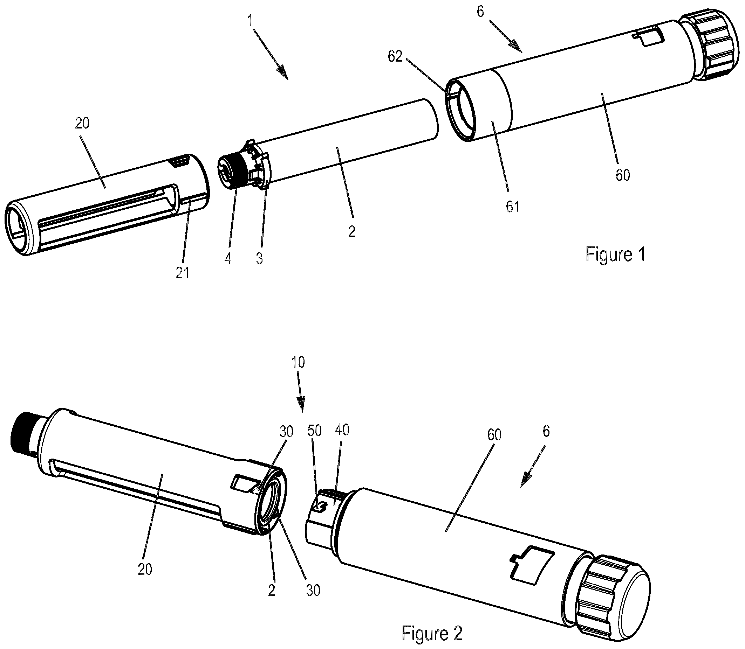

Figure 1 depicts a perspective view of an injection pen 1 comprising a lock mechanism 10 according to the present invention. The injection pen 1 includes a proximal part 6 accommodating a dose and dispensing mechanism for injection a liquid drug from a reservoir, e.g. a cartridge 2. Furthermore, the injection pen 1 includes a reservoir holder or cartridge holder 20 holding the cartridge 2 in place relative to a housing 60 and releasably attachable and lockable to the proximal part 6.

In the embodiment shown, the cartridge 2 comprises a coding ring 3 ensuring that the cartridge 2 can exclusively be connected to the cartridge holder if a coding feature of the ring 3 matches a counter coding features in the cartridge holder 20. The coding mechanism is not linked to or required by the lock mechanism 10 described in the following. That means in a second embodiment the injection pen 1 may not comprise a coding mechanism and the cartridge 2 may be held in the cartridge holder 20 without coding ring and coding feature.

Figure 2 depicts a perspective view of the cartridge holder 20 in a disconnected state. In figure 2 the housing 60 is depicted without a distal ring-shaped housing connecting member 61 (shown in figure 1) such that a lock member 40 of the injection pen can be seen. In order to insert a cartridge 2 or to replace a cartridge the cartridge holder 20 can be detached from the proximal part 6. The cartridge holder 20 is releasably connectable to the proximal part 6 by insertion of a proximal end of the cartridge holder 20 into the housing connecting member 61.

In the following the details of the structural features of the lock mechanism 10 according to the invention are described in detail. Subsequently, the function of the lock mechanism 10 is described.

As best shown in figure 1 and 2 the cartridge holder 20 is sleeve shaped with an opening adapted to house the cartridge 2. The coding ring 3 includes a needle mounting portion adapted to be releasably connected to a needle assembly. Alternatively, if a standard cartridge holder is used without coding ring the cartridge holder comprises the needle mounting portion. In a proximal end portion, the cartridge holder 20 includes a coupling portion including on an outside two oppositely arranged longitudinal guiding elements in form of guiding ribs 21 extending along the longitudinal axis (figure 1).

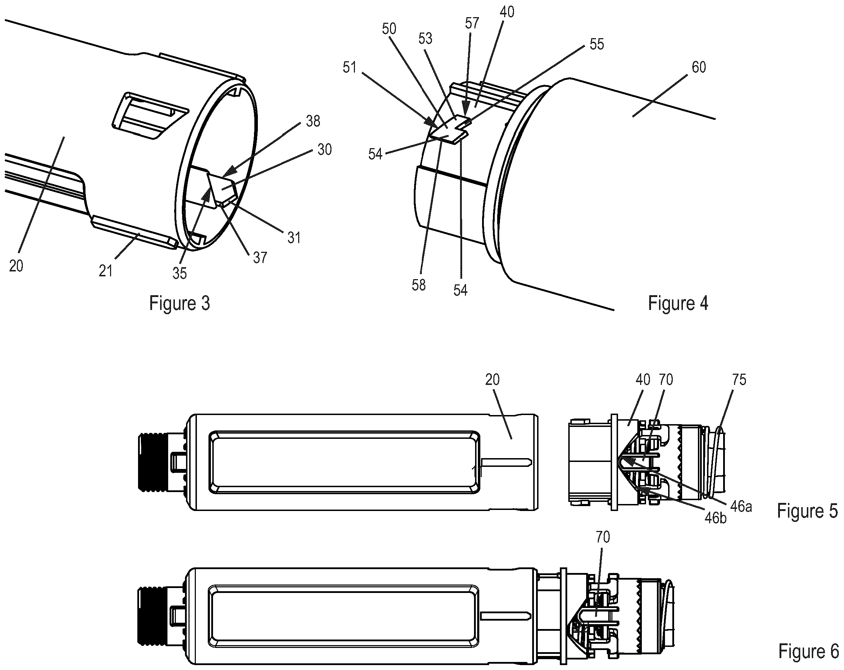

Figure 3 depicts a perspective view of a distal end section of the cartridge holder 20. On an inner side in the distal end section the cartridge holder 20 includes two oppositely arranged radially inwards extending holder protrusions 30. On a proximal side each protrusion 30 comprises a first sloped surface 31 which is proximally directed and on a distal side the protrusion comprises a first stop surface 35 which is sloped too with respect to the longitudinal axis and distally directed. Laterally, between the first sloped surface 31 and the first stop surface 35 each protrusion comprises on a first side a first axial surface 37 and on an opposite side a third axial surface 38. The two axial surfaces 37, 38 are parallel to the longitudinal axis of the cartridge holder 20 and the housing 60.

The housing 60 is sleeve-shaped and houses the dose and dispensing mechanism of the injection pen 1. In a distal end portion, the ring-shaped connecting member 61 (see figure 1) is located having the same outer diameter as the proximal housing. As best shown in figure 1, on an inside of the connecting member 61 two oppositely longitudinal guiding elements in form of a longitudinal grooves 62 are arranged and adapted to accommodate the guiding ribs 21 of the cartridge holder 20 to rotationally couple the cartridge holder 20 but allowing a relative axial movement during attachment.

Figure 4 depicts a perspective view of a distal end portion of the housing 60 without the connecting member 61. As shown in figure 4 a sleeve-shaped lock member 40 positioned inside the connecting member 61. The lock member 40 is rotationally guided within the housing by a circumferentially extending rib which is located in a corresponding groove in the housing (both not shown). Therefore, the lock member 40 can rotate but is prevented from being moved axially relative to the housing 60 and relative to the connecting member 61.

As it can be seen in figure 4, on its outside the lock member 40 includes two oppositely arranged lock protrusion 50 each having a L-shaped geometry. A first portion or leg 53 of the L-shaped protrusion extends circumferentially and comprises on a distal side a distally directed second sloped surface 51 and on a proximal side a proximally directed second stop surface 55 which is sloped too with respect to the longitudinal axis. On a lateral side the first portion includes a second axial surface 57 which is parallel to the longitudinal axis.

A second portion or leg 54 of the L-shaped protrusion 50 extends along the longitudinal axis and comprises on a proximally directed face a release surface 59 and on a lateral side a fourth axial surface 58.

Figures 5 and 6 depict a side view of the reservoir holder with a cartridge and a distal portion of the lock mechanism without housing 60 and without connecting member 61. As shown in these figures the lock member 40 has at a proximal end two oppositely arranged pairs of angled surfaces 46a, 46b forming an arrow-shaped recess.

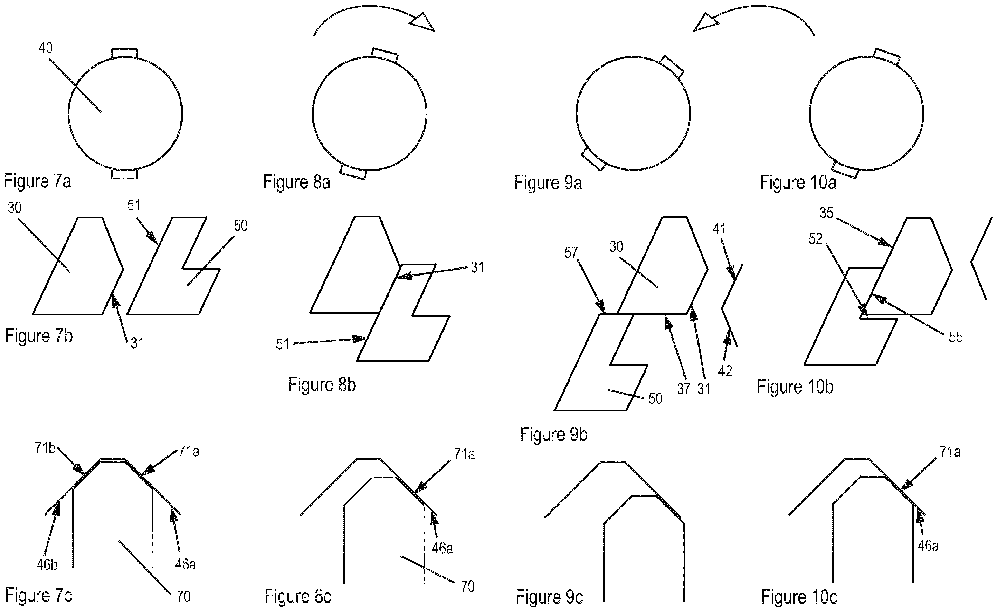

A cam 70 is positioned inside the recess and can be moved exclusively along the longitudinal direction and is prevented from rotating by an axial guide. The cam 70 has an arrow-shaped distal end with two angled counter surfaces 71 (shown in figure 7c) matching the two angled surfaces 46a, 46b of the lock member recess. The longitudinally movable cam 70 is biased in distal direction by a mechanical spring 75 such that at least one of the cam surfaces 71a, 71b (figure 7c) is pressed against an angled surface 46a, 46b of the lock member 40. As the lock member 40 cannot move axially but only rotate the lock member 40 is biased in a start position. That means the spring force must be overcome to rotate the lock member 40 out of its start position.

In the following the attachment process to attach and lock the cartridge holder 20 to the proximal part 6 of the injection pen 1 and the detaching process to release the cartridge holder 20 from the proximal part is descripted in detail with respect to the figures 7 to 14. Those figures depict schematical views of the lock member 40 (figures 7a to 14a), the holder protrusion 30 and the lock protrusion 50 (figures 7b to 14b) and the position of the cam 70 relative to the angled surfaces 46a, 46b of the lock member (figure 7c to 14c).

To attach and lock the cartridge holder 20 to the proximal part 6 the user inserts the proximal end of the cartridge holder 20 into the ring-shaped connection member 61 of the housing 60 such that the cartridge holder end is radially in between an outer surface of the lock member 40 and an inner surface of the connection member 61. For the insertion, the cartridge holder 20 has to be rotationally aligned to the connecting member 61 such that the cartridge holder guiding ribs 21 can enter the longitudinal grooves 62. The cartridge holder 20 is then rotationally coupled to the housing 60. This state is shown in figure 6.

Subsequently, the user can move the cartridge holder 20 proximally towards an attached position. Thereby, the first and second sloped surfaces 31, 51 are moved towards each other and get into contact. Figures 7a, 7b and 7c depict schematical views representing a non-connected state when the lock member 40 is in its non-rotated start position and the cartridge holder 20 is rotationally aligned to the lock member and the holder protrusion 30 is in front of the lock protrusion 50.

When the cartridge holder 20 is further moved proximally the first and second sloped surfaces 31, 51 are pressed against each other as schematically depicted in figure 8b. As the lock member 40 cannot move axially the lock member 40 is forced to rotate against the spring force such that the first and second sloped surfaces 31, 51 slide along each other thereby rotating the lock member 40 out of its start position towards a rotated (offside) position. The lock member 40 is rotated in clockwise direction seen from distal to proximal, schematically depicted in figure 8a. During the lock member rotation, the lock member angled surface 46a is pressed against the angled cam surface 71a which is depicted in figure 8c. As the cam 70 cannot rotate the cam is forced to move proximally against the spring force of the mechanical spring 75. That means the lock member 40 is rotated against the spring force. During rotation the mechanical spring 75 is further compressed.

The lock member 40 is rotated as long as the sloped surfaces 31, 51 slide along each other. When the first surface 31 has passed the second sloped surface 51 and when the cartridge holder is further moved proximally relative to the housing and relative to the lock member 40 the lock protrusion 50 is laterally and adjacent to the holder protrusion 30 (as shown in figure 9b). Figure 9a depicts the rotated lock member 40 in its rotated offside position such that the holder protrusion 30 can pass the lock protrusion 50 in proximal direction. Figure 9c depicts the position of the cam 70 in the lock member recess in this state.

During further proximal movement, the first axial surface 37 of the holder protrusion 30 slides along the second axial 57 surface of the lock protrusion 50 (state as shown in figure 9b). As soon as the first axial surface 37 has passed the second axial surface 57 the holder protrusion 30 is positioned proximal to the lock protrusion 50 and the lock member 40 is free to rotate back. That means the lock member 40 is no longer prevented from being rotated towards its start position by the spring force of the biased spring 75.. The lock member 40 thus rotates in counterclockwise direction (figure 10a) as the cam surface 71a is pressed against the angled surface 46a (figure 10c).

The lock member 40 includes in a proximal portion a first restriction surface 41 and a second restriction surface 42 which are adjacent and form an arrowhead-shaped geometry (figure 9b). The first restriction surface 41 has the same slope as the first sloped surface 31 and the second restriction surface has the same sloped as a back surface 39 of the holder protrusion, see figure 9b. The restriction surfaces are adapted to engage the holder protrusion and to restrict an axial travel of the holder protrusion. That means if the user keeps moving the cartridge holder 20 in proximal direction the first sloped surface 31 contacts and abuts the first sloped restriction surface 41 and thus further axial movement is prevented. Due to its slope the first restriction surface 41 guides the protrusion 30 towards a holding position.

As the holder protrusion 30 is then proximal to the lock protrusion 50 the second stop surface 55 of the lock protrusion 50 is moved in front of the first stop surface 35 and the lock protrusion 50 moves such that the holder protrusion 30 enters into a region of a crook of the L-shaped lock protrusion 50, as best shown figure 10b. The lock member 40 is then in a rotated holding position.

As the first and second stop surfaces 35, 55 are sloped with respect to a direction perpendicular to the longitudinal axis the holder protrusion 30 and the lock protrusion 50 are safely held in place by the sloped stop surfaces 35, 55 which may contact and engage each other. That means in this state when the cartridge holder 20 is pulled in distal direction away from the attached position the stop surfaces 35, 55 slide along each other until the holder protrusion 30 abuts the second stop surface 55 and a lateral wall 52 (figure 10b) in the crook of the L-shaped protrusion. The cartridge holder 20 is then its attached position and held in place and cannot be pulled out of the housing in distal direction.

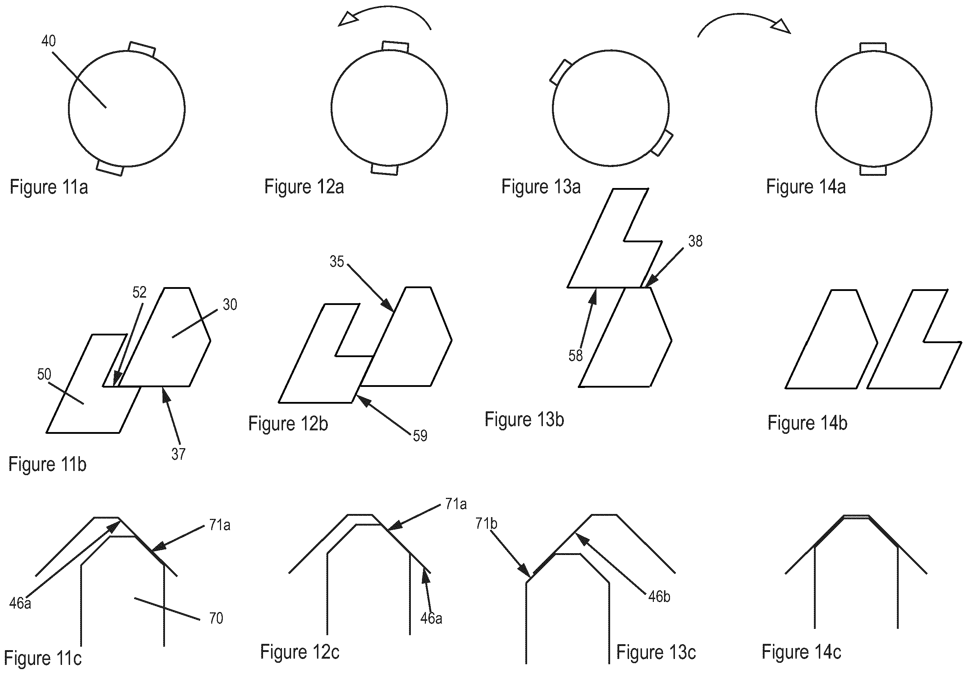

In the following the release and detaching of the cartridge holder is described in detail with respect to schematic figures 11 to 14.

To unlock and detach the cartridge holder 20 from the proximal part 6 the user has to push the cartridge holder 20 a short distance from the locked position further proximally relative to the lock member 40 and relative to the housing 60 into proximal end position. The lock member 40 does not rotate (figure 11a) and remains in its rotated holding position. The proximal movement causes the holder protrusion 50 to move out of crook region as shown in figure 11b. As the lock member 40 does not rotate the biasing by the cam remains constant, figure 11c.

The second restriction surface 42 of the lock member 40 restricts proximal movement of the cartridge holder relative to the lock member and housing (figure 11b). That means the sloped back surface 39 of the holder may engage the sloped second surface 42 and due to the slope the second restriction surface 42 guides the protrusion 30 towards a release path as shown in figures 12b and 13b.

The holder protrusion 30 moves proximal relative to the lock protrusion 50 until the first axial surface 37 has passed the lateral wall 52 of the lock protrusion 50. The lock member 40 is then free to rotate in counterclockwise direction out of its holding position. This state is shown in figures 12a, 12b and 12c. The lock member is rotated by the biased spring as the cam surface 71a is pressed against the lock member angled surface 46a.

As in this state, the cam surface 71a of the biased cam 70 still presses against the angled surface 46a (figure 12c) the lock member is forced to rotate counterclockwise (figure 12a). The distally directed sloped stop surface 35 of the holder protrusion and the proximally directed sloped release surface 59 of the lock protrusion 50 are pressed against each other (figure 12b) and as a consequence slide along each other. The lock member is thus further rotated in counterclockwise direction.

When the user further pulls the cartridge holder distally out of the housing the sloped stop surface 35 and the release surface 59 are pressed against each other and further rotate the lock member 40 in counterclockwise direction. That causes the angled surface 46b to be pressed against the cam surface 71b (figure 13c) and further distal movement of the cartridge holder thus pushes the cam 70 proximally against the spring force.

When the lock member 40 is in a rotated release position (figure 13a) the two surfaces are slid along each other and the lock protrusion 50 can pass the holder protrusion 30 and is adjacent the holder protrusion 30 as shown in figure 13b. That means during release the holder protrusion 30 is on the opposite side of the lock protrusion 50 than during attachment.

In the state shown in figures 13a to 13c, the third axial surface 38 of the holder protrusion 30 slides along the fourth axial surface 58 of the lock protrusion 50. The cartridge holder 20 can thus be moved further distally and away from the proximal part 6. When the holder protrusion 30 has passed the lock protrusion 50 (the third axial surfaced has passed the fourth axial surface) the lock member 40 is moved back into its non-rotated start position as the cam surface 71b is pressed against the angled surface 46b by the biased spring. Subsequently, the cartridge holder 20 can be completely removed from proximal part 6. This state is shown in figures 14a, 14b and 14c.

As the lock member 40 is again in its initial non-rotated position the lock mechanism is ready to re-connect the cartridge holder 30 with a new cartridge to the housing.

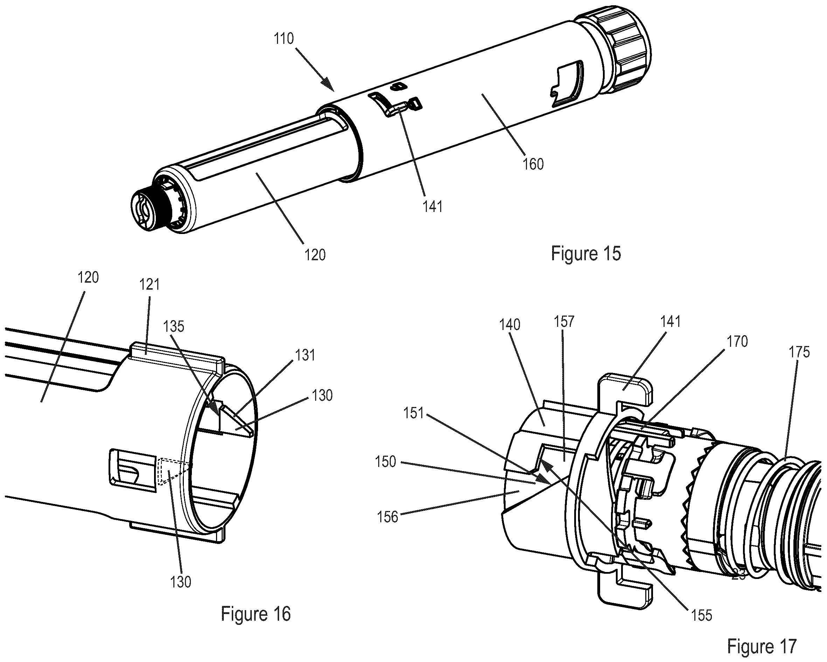

Figure 15 depicts a perspective view of an injection pen 100 with a second embodiment of the lock mechanism 110 according to the invention.

As in the first embodiment described above the injection pen 100 according to the second embodiment includes a cartridge holder 120 that can be releasably attached and locked to a proximal part 106 of the injection pen 100. In contrast to the first embodiment, to release the cartridge holder 120 from the proximal part 106 the lock member 140 can be rotated by the user by a user actuating element in form of a lever 141. The lever 141 is fixedly connected to the lock member 140 and thus a rotation of the lever 141 rotates the lock member 140. In the following the structural features of the second embodiment and subsequently, the function are described in detail.

Figure 16 depicts a perspective view of a proximal end portion of the cartridge holder 120. The cartridge holder 120 includes two oppositely arranged longitudinal guiding ribs 121 that can engage corresponding grooves in the housing (not shown). On an inside of the sleeve-shaped cartridge holder 120 two oppositely arranged holder protrusions 130 are arranged. Each protrusion 130 includes on a proximal face a proximally directed first sloped surface 131 and on a distal face a distally directed first stop surface 135.

Figure 17 depicts a distal end portion of the proximal part 106 without the outer housing. The lock member 140 is rotationally guided by a support structure and the housing such that the lock member 140 can rotate but is prevented from being moved longitudinally relative to the housing 160. On its outer surface the lock member 140 includes two oppositely arranged guiding grooves 150 adapted to accommodate and guide the holder protrusion 130. Each guiding groove 150 comprises a sloped section 156 and an end section 157 connected to the sloped section. The sloped section 156 comprises a distally oriented second sloped surface 151 and the end section 157 includes a proximally oriented second stop surface 155.

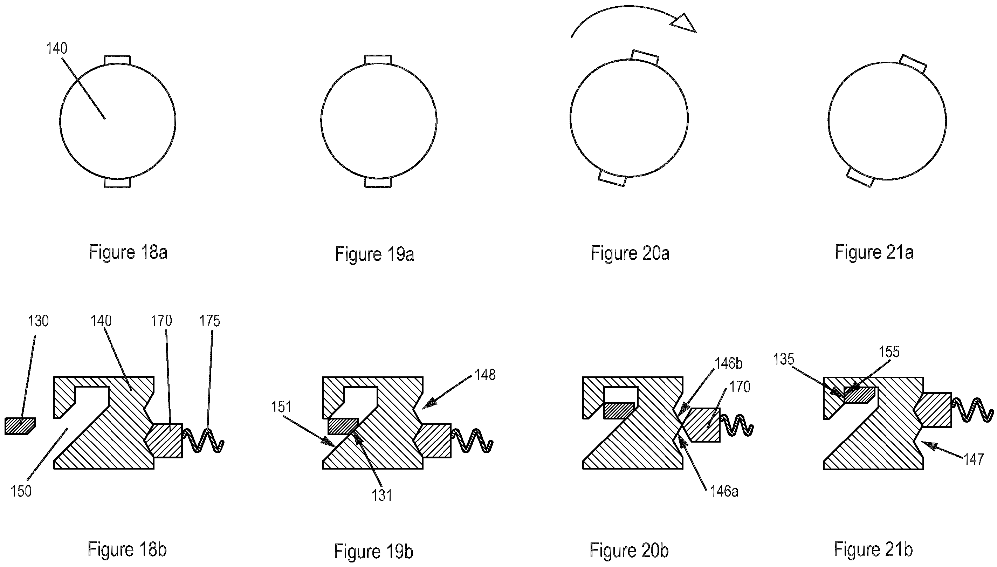

Furthermore, the lock member 140 comprises a recess formed by angled surfaces 146a, 146b (figure 20b) and a cam 170 biased by a spring 175 which biases the lock member into a start position as described in detail above with respect to the first embodiment.

In the following the attachment process to attach and lock the cartridge holder 120 to the proximal part 106 and the detaching process to release the cartridge holder 120 for the second embodiment is descripted in detail with respect to the figures 18 to 21. Those figures depict schematical views of the lock member 140 (figures 18a to 21a) and the holder protrusion 130 with guiding groove 150 and the cam 170 (figures 18b to 21b).

To attach the cartridge holder 120 to the proximal part 106 of the injection pen the user inserts the proximal cartridge holder end into the housing 160. As depicted in figures 18a and 18b the holder protrusion 130 is not yet inside the guiding groove 150 and the lock member 140 is in a non-rotated start position. As the cam 170 is pressed by the spring 175 inside the arrowhead-shaped recess the lock member is in a stable position.

When the cartridge holder 120 is further moved proximally relative to the housing 160, the rib 121 enters the longitudinal groove in the housing and thus rotationally couples the cartridge holder 120 to the housing. Furthermore, the holder protrusions 130 enters the guiding grooves 150 and the first sloped surface 131 gets into contact with the second sloped surface 151, see figure 19b. The lock member 140 is in the start position (figure 19a).

Further proximal movement causes the first and second sloped surfaces 131, 151 to slide along each other thereby rotating the lock member 140 in clockwise direction. As a lock member angled surface 146a is pressed against the biased cam 170 the rotation is against a spring force of a spring 175. Figure 20a depicts the rotated lock member 140 and figure 20b depicts the state where the holder protrusion 130 is at an end of the sloped section 156 of the guiding groove 150. As it can be seen in figure 20b the cam 170 is on a top of the two adjacent recesses. When the cartridge holder 120 is further moved proximally and thus the lock member 140 is further rotated the cam surface engages a opposite angled surface 146b of the lock member. From this tipping point and due to the biased cam 170 the lock member 140 is rotated further in clockwise direction until the holder protrusion 130 is in the end section 157 of the groove 150 as shown in figures 21b. The lock member 140 is then in a stable rotated position as the cam 170 is in a second recess of the lock member.

The first stop surface 135 is then proximally located to the second stop 155 surface and the two stop surfaces 135, 155 are rotationally aligned such that they can engage each other. Differently stated, when the user pulls the cartridge holder 120 in distal direction the first stop surface 135 is pressed against the second stop surface 155 thus locking the cartridge holder to the proximal part and the cartridge holder is in its attached and locked position.

To release and detach the cartridge holder 120 from the proximal part 106 the user can rotate the actuation lever 141 in counterclockwise direction which causes rotation of the lock member 140 and the holder protrusion 130 to leave the end section 157 of the guiding groove 150 and subsequently the first and second sloped surface 131, 151 re-engage and slide along each other which in turn causes the cartridge holder to be moved distally away from the proximal part 106.

When the top of the cam 170 has passed the tripping point between two adjacent recesses and the cam presses against the angled surface 146a and the lock member 140 is further rotated towards its start position by the spring force until the cam is positioned in the recess as in the start position shown in figure 18b. The holder protrusion 130 is then no longer inside the guiding groove and the cartridge holder 120 can be completely removed from the proximal part 106. The lock mechanism is then ready to re-connect the cartridge holder 120 to the proximal part 106.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

**LIST OF DESIGNATIONS**

| | | | | | |
|---|---|---|---|---|---|
| 1 | Injection pen | 40 | lock member | 100 | injection pen |
| 2 | cartridge | 41 | first restriction surface | 106 | proximal part |
| 3 | coding ring | 42 | second restriction surface | 110 | lock mechanism |
| 4 | needle mounting portion | 46a | angled surface | 120 | cartridge holder |
| 6 | proximal part | 46b | angled surface | 121 | guiding rib |
| | | | | 130 | holder protrusion |
| 10 | lock mechanism | 50 | lock protrusion | 131 | first sloped surface |
| 20 | cartridge holder | 51 | second sloped surface | 135 | first stop surface |
| 21 | guiding ribs | 52 | lateral wall | | |
| | | 53 | first portion | 140 | lock member |
| 30 | holder protrusions | 54 | second portion | 141 | actuation lever |
| 31 | first sloped surface | 55 | second stop surface | 146a | angled surface |
| 35 | first stop surface | 57 | second axial surface | 146b | angled surface |
| 37 | first axial surface | 58 | fourth axial surface | 147 | first recess |
| 38 | third axial surface | 59 | release surface | 148 | second recess |
| 39 | back surface | | | | |
| | | 60 | housing | 150 | guiding groove |
| | | 61 | connecting member | 151 | second sloped surface |
| | | 62 | longitudinal grooves | 155 | second stop surface |
| | | | | 156 | sloped section |
| | | 70 | cam | 157 | end section |
| | | 71a | angled cam surfaces | | |
| | | 71b | angled cam surface | 160 | housing |
| | | 75 | spring | 170 | cam |
| | | | | 175 | spring |

## Claims

1. Lock mechanism (10) for locking a distal reservoir holder (20) to a proximal part (6) of an injection device (1) for dispensing a liquid drug from a reservoir (2), the lock mechanism (10) comprising
- the distal reservoir holder (20) adapted to hold the reservoir (2) in place relative to the proximal part (6) of the injection device (1) and releasably attachable and lockable to the proximal part (6), the reservoir holder (20) comprising
- a first longitudinal guiding element (21);
- a proximally directed first sloped surface (31);
- a distally directed first stop surface (35);
- the proximal part (6) defining a longitudinal axis and comprising
- a housing (60) adapted to accommodate a dose and dispensing mechanism for dispensing the liquid drug;
- a spring member (75);
- a second longitudinal guiding element (62);
- a lock member (40) rotationally guided such that a rotation of the lock member (40) relative to the housing (60) is allowed but a longitudinal movement is prevented, the lock member (40) comprising
i. a distally directed second sloped surface (51) and
ii. a proximally directed second stop surface (55);
wherein during an attachment movement of the reservoir holder (20) relative to the proximal part (6) in proximal direction towards a locked position to attach and lock the reservoir holder (20) to the proximal part (6)
- the first and second guiding element (21, 62) are coupled to each other thereby allowing a longitudinal movement but preventing a rotation of the reservoir holder (20) relative to the housing (6);
- the first and second sloped surface (31, 51) are pressed against each other and subsequently slide along each other thereby causing the lock member (40) to rotate relative to the housing (60) from a start position towards a rotated position;
- the first stop surface (35) is distally to the second stop surface (55);
wherein when the reservoir holder (20) is in its attached and locked position
- the lock member (40) is in the rotated position, wherein lock member rotation allows the first stop surface (35) to pass the second stop surface (55) such that in the attached and locked position the first stop surface (35) is located proximal to the second stop surface (55) and
- the lock member (40) is biased in its rotated position by the spring member (75);
- wherein due to the lock member rotation the first and second stop surface (35, 55) are rotationally aligned to each other and can cooperate with each other thereby preventing a longitudinal movement of the reservoir holder (20) in distal direction and locking the reservoir holder (20) to the proximal part (6).

2. Lock mechanism (10) according to claim 1, wherein the reservoir holder (20) includes a holder protrusion (30) comprising on a proximal side the first sloped surface (31) and on a distal side the first stop surface (35).

3. Lock mechanism (10) according to claim 1 or 2, wherein the rotation of the lock member (40) from its start position towards the rotated position is against a spring force of the spring (75).

4. Lock mechanism (10) according to claim 3, further including a cam (70) guided by a longitudinal guide and longitudinally movable and comprising an angled surface (71a, 71b) and wherein the lock member (40) comprises an angled counter surface (46a, 46b) wherein the cam angled surface (71a, 71b) is pressed against the angled counter surface (46a, 46b) thereby biasing the lock member (40) in its start position.

5. Lock mechanism (10) according to any of claims 1 to 4, wherein the first and the second stop surfaces (35, 55) are sloped with respect to a direction perpendicular to the longitudinal axis.

6. Lock mechanism (10) according to any of claims 1 to 5, wherein the lock member (140) includes a guiding groove (150) comprising in a distally faced wall the second sloped surface (151) and on a proximally faced wall the second stop surface (155).

7. Lock mechanism (10) according to any of claims 1 to 6, wherein the lock member (140) includes a user actuating element (141) which can be moved by the user to rotate the lock member back or to rotate the lock member out of its rotated position.

8. Lock mechanism (10) according to claim any of claims 1 to 5 or claim 10, wherein the lock member (40) includes a lock protrusion (50) comprising on a distal side the second sloped surface (51) and on a proximal side the second stop surface (55).

9. Lock mechanism (10) according to claim 8, wherein the lock protrusion (50) is L-shaped having a first portion (53) extending longitudinally and comprising the second axial surface (57) and a second portion (54) extending circumferentially around the longitudinal axis and including the second sloped surface (51) and the second stop surface (55).

10. Lock mechanism (10) according to any of claims 8 or 9, wherein the reservoir holder (20) further includes a first axial surface (37) oriented parallel to the longitudinal axis and the lock member (40) further includes a second axial surface (57) oriented parallel to the longitudinal axis, wherein when the lock member (40) is in its rotated position the first and second axial surfaces (37, 57) cooperate with each other such that a further longitudinal movement of the reservoir holder (20) relative to the lock member (40) is allowed but a return rotation of the lock member (40) towards its initial position is prevented by the axial surfaces.

11. Lock mechanism (10) according to claim 10, wherein when the reservoir holder (20) is in its locked position the first and second axial surfaces (37, 57) have passed each other and are separated from each other in the longitudinal direction such that the lock member (40) can be rotated towards the start position or into the holding position

12. Lock mechanism (10) according to any of claims 8 to 11, wherein when the reservoir holder (20) is in its locked position the first and second sloped surfaces (31, 51) have passed each other and the lock member is rotated back into its start position or into a holding position such that the first and second stop surface (35, 55) are rotationally aligned to each other and cooperate with each other thereby preventing a longitudinal movement of the reservoir holder (20) in distal direction and locking the reservoir holder (20) to the housing (60, 61).

13. Lock mechanism (10) according to any of claims 1 to 12, wherein the housing (60, 61) is sleeve-shaped and the reservoir holder (20) is sleeve-shaped with an outer diameter smaller than the housing such that the reservoir holder (20) can be inserted into the housing (60, 61) to attach the reservoir holder (20) to the housing.

14. An injection device (1) comprising the lock mechanism (10) according to any of claims 1 to 13, wherein the injection device (1) comprising a dose and dispensing mechanism accommodated inside the housing (60, 61).

15. Method for attaching and subsequently locking a reservoir holder (20) to a proximal part (6) of an injection device (1), the method comprises the steps of
a) Inserting a proximal end portion of the reservoir holder (20) into a distal end portion of the proximal part (6) which longitudinally guides the reservoir holder (20) and prevents a rotation of the reservoir holder relative to the proximal part (6);
b) Moving the reservoir holder (20) proximally relative to the proximal part towards a locked position;
c) Pressing, by the proximal movement, a reservoir holder (20) first sloped surface (31) against a lock member second sloped surface (51);
d) Rotating, by the sloped surfaces engagement, the lock member (40) relative to a housing (60) of the proximal part into a rotated position;
e) Biasing the lock member into the rotated position by a spring;
f) Moving, due to the rotation, a reservoir holder first stop surface (35) proximally to a lock member second stop surface (55);
g) Rotationally aligning, by the movement, the first stop surface (35) and the second stop surface (55) thereby preventing a longitudinal movement of the reservoir holder in distal direction and locking the reservoir holder to the proximal part (6).
